(19) Europäisches Patentamt

European Patent Office

Office européen des brevets

(11) Veröffentlichungsnummer: **0 609 687 A1**

(12) **EUROPÄISCHE PATENTANMELDUNG**

(21) Anmeldenummer: **94100604.1**

(51) Int. Cl.5: **C02F 1/52**

(22) Anmeldetag: **17.01.94**

(30) Priorität: **02.02.93 DE 4302910**

(43) Veröffentlichungstag der Anmeldung:
**10.08.94 Patentblatt 94/32**

(84) Benannte Vertragsstaaten:
**BE CH FR GB IT LI NL**

(71) Anmelder: **Straten, Günter**
**Grossheidstrasse 228**
**D-52080 Aachen(DE)**

(72) Erfinder: **Straten, Günter**
**Grossheidstrasse 228**
**D-52080 Aachen(DE)**

(74) Vertreter: **Müller-Boré & Partner**
**Patentanwälte**
**Isartorplatz 6**
**D-80331 München (DE)**

(54) **Fällmittel zur Ausfällung von Schwermetallen.**

(57) Beschrieben wird ein Fällmittel mit Thioacetamid zur Ausfällung von Schwermetallen aus Abgasen bzw. Abwässern, wobei es Thioacetamid ($C_2H_5SN$) in einer 1-20 %igen wäßrigen Lösung enthält, die zusätzlich eine Puffersubstanz zur Verhinderung des hydrolytischen Selbstzerfalls enthält, wobei die Lösung einen pH-Wert zwischen 5,5 und 9,0 aufweist.

EP 0 609 687 A1

Die vorliegende Erfindung betrifft ein Fällmittel mit Thioacetamid zur Ausfällung von Schwermetallen aus Abgasen bzw. Abwässern, ein Verfahren zu dessen Herstellung sowie dessen Verwendung.

Das Fällmittel dient insbesondere zur Schwermetallentfernung aus Abgasen und Gaswäschern von Kraftwerken mit fossilen Brennstoffen und von Müllverbrennungsanlagen. Das Mittel dient ferner zur Fällung von Schwermetallen aus Abwässern von Verbrennungsanlagen und Müllverbrennungsanlagen sowie aus anderen schwermetallbelasteten Abwässern.

Aus der DE 39 07 066 C2 ist bereits die Verwendung von Thioacetamid zur Dekontaminierung von schlammartigen Sedimenten bekannt. Ferner ist aus der DE-OS 26 28 649 bereits ein Verfahren zur Abtrennung von Schwermetall-Ionen aus wässrigen Lösungen von Komplex-Verbindungen bekannt, bei dem Salze von Monothio-Carbonsäuren verwendet werden, gegebenenfalls in Anwesenheit eines Phosphat-Puffers.

Aus der EP 0 043 759 A1 ist bereits ein hydrophiles Substrat zur Absorption von Schwermetallen bekannt, bei dem auch die Ausfällung von Metallsulfiden durch eine wäßrige Lösung von Natriumsulfit, Kaliumsulfit, Ammoniumsulfit oder Thioacetamid zum Einsatz kommt.

Ferner ist aus der EP 0 333 672 A1 bereits ein langsam lösliches Festkörperprodukt bekannt, das aus Substanzen gebildet ist, die geeignet sind, Verunreinigungen in den Abwässern einer zahnärztlichen Einrichtung auszufällen. Es wird unter anderem vorgeschlagen, eine organische Verbindung, z.B. Thioacetamid zu verwenden, welche Schwefelwasserstoff in der Hydrolyse erzeugt.

Bei Kraftwerksanlagen unter Einsatz fossiler Brennstoffe und bei Müllverbrennungsanlagen, die die Rauchgase nach dem Prinzip der Naßwäsche entschwefeln und entsticken, entsteht ein Abgas mit zum Teil erheblichen Konzentrationen an flüchtigen Schwermetallen, beispielsweise Arsen (As) Antimon (Sb), Quecksilber (Hg), Cadmium (Cd), Blei (Pb), Zinn (Sn), Zink (Zn) u.a.

Insbesondere die Quecksilberbelastung der Abgase bereitet aus verschiedenen Gründen große Probleme. Eine weitere Schwierigkeit stellt die Arsenbelastung bei der Verfeuerung von Braunkohle, Steinkohle und Schweröl dar. Das Arsen ist zum Teil in erheblichen Mengen in vielen Kohlearten und einigen Schwerölen enthalten und gelangt bei deren Verfeuerung zwangsläufig auch in die Rauchgase, und zwar in gasförmigem Zustand.

Die Entschwefelung und Entstickung von Abgasen aus den vorstehend genannten Anlagen geschieht herkömmlich auf zwei grundsätzlich verschiedene Arten, nämlich durch ein sogenanntes Trockenwaschverfahren und ein sogenanntes Naßwaschverfahren.

Die vorliegende Erfindung dient insbesondere zum Einsatz beim Naßwaschverfahren von Abgasen aus Müllverbrennungsanlagen und liefert eine spezielle Lösung für die Problem- und Aufgabenstellung in diesem Bereich.

Verbrennungsabgase aus Kraftwerken, die fossile Brennstoffe verfeuern, sowie aus Müllverbrennungsanlagen führen nach dem Durchgang durch Elektrofilter und andere Flugstaub- und Partikelabscheider neben Feinstfraktionen von Partikeln auch gasförmige Bestandteile mit sich. Ein zentrales Problem stellen hierbei flüchtige Schwermetalle dar, insbesondere Arsen und Quecksilber. Von den eingangs genannten Metallen lassen sich alle mit Ausnahme von Arsen und Quecksilber ohne Schwierigkeiten allein durch Kontakt mit Wasser nahezu vollständig aus dem Gasstrom entfernen.

Bei Quecksilber und Arsen gelingt dies nur unvollständig, was einerseits messbare Restgehalte dieser Metalle im Reingas verursacht und andererseits bei mehrstufigen Waschverfahren zu einer Verschleppung dieser Stoffe in Folgestufen führt. Bei mehrstufigen Abgaswaschanlagen wird die erste Stufe meist als "saure Waschstufe" mit Frischwasserdurchlauf oder auch im Kreislaufstrom betrieben, um die genannten Schwermetalle, Salzsäure und andere Verunreinigungen zurückzuhalten. Die zweite Stufe arbeitet entweder direkt mit Kalkmilch (Emulsion von $Ca(OH)_2$ in Wasser) oder häufiger mit Natronlauge, um Schwefeldioxid aus dem Abgas zu entfernen. Bei Verwendung von Natriumhydroxid entsteht Natriumsulfat ($Na_2SO_4$), das anschließend in einer "Gipsstufe" mit Calciumchlorid ($CaCl_2$) zu Gips ($CaSo_4$) und Natriumchlorid (NaCl) umgesalzen wird.

Wenn Arsen oder Quecksilber in diese $SO_2$-Waschstufe gelangen, führt dies zwangsläufig zur unerwünschten Verunreinigung des Gipses, was dessen Verwendung einschränkt oder gar ausschließt. Gleichzeitig verbleiben meßbare Restgehalte der Metalle in dem gereinigten Abgas und gelangen somit in die Außenluft.

Die weitgehende Beseitigung des Arsens im Abgas aus sauren Waschstufen von zweistufigen Waschanlagen läßt sich durch die Zugabe von entsprechenden Arsen-bindenden Stoffen meist ohne Schwierigkeiten lösen. Erheblich problematischer ist die Metallentfernung, wenn Arsen und Quecksilber oder nur Quecksilber als nennenswerte Verunreinigungen im Abgas vorhanden sind, da das Quecksilber in Folge der stark reduzierend wirkenden Matrix (vorrangig $SO_2$) in der heißen sauren Waschzone des ersten Wäschers in instabilen Übergangszuständen von $Hg^{I}Hg^{II}$ vorliegt. Dieses Hg gelangt durch die Sprühwäsche in überwiegend gelöster Form (größer 90 %) aber nur als Teil der Gesamtmasse in die wäßrige saure Phase, wobei das Gas von zuvor 180° C bis 230° C auf ca. 150° C abkühlt. Das $SO_2$ bleibt aufgrund des stark-salzsauren pH-Wertes (0,2 - 1,5) weitgehend im Abgas gelöst.

Bei sauren Waschstufen mit Kreislaufwasserführung wird nur ein geringer Teilstrom pro Stunde als Absalzung und Abschlammung des Wäschersumpfes durch Frischwasser ersetzt, was zu einer Anreicherung des gelösten Quecksilbers im Waschwasser führt. Als Folge davon stellt sich ein Quecksilbergleichgewicht in der Gas-Dampf-Wasser-Phase ein, das von den Faktoren Hg-Gehalt im Rohgas, Hg-Gehalt in der Waschlösung, Temperatur des Systems, Druck des Systems, pH-Wert u.a. bestimmt wird.

Nach den Gesetzen der physikalischen Chemie ist daher eine weitgehende oder gar vollständige Elimination des Quecksilbers aus dem Abgas in dieser Verfahrensführung nicht erreichbar. Eine Ausfällung des Hg als Quecksilbersulfid (HgS) scheitert an der stark reduzierenden Wirkung des bei der Anwendung polysulfidischer Mittel in saurer Lösung entstehenden Schwefelwasserstoffs ($H_2S$), der eine partielle Disproportionierung des Quecksilbers zu $Hg^{II}$ und $Hg^0$ bewirkt.

Bei Anwendung anorganischer sulfidischer und polysulfidischer Stoffe entstehen je nach Anwendungsmenge des Fällungsmittels zwischen 5 % bis 30 % des enthaltenen Quecksilbers als elementares Quecksilber ($Hg^0$), das vom Abgas je nach Temperatur gemäß seinem Partialdruck mitgenommen und so in die $SO_2$-Waschstufe verschleppt wird und dort entweder in den Gips gelangt oder teilweise über das Reingas wieder in die Außenluft verbracht wird.

Die Anwendung organischer Schwefelverbindungen scheidet meist aus, da diese in saurer Lösung in der Regel nur gering oder überhaupt nicht bindig sind und organische Schwefel-Quecksilberaddukte bilden, die in der heißen Abgaszone das Quecksilber thermisch wieder abspalten. Die Grenzwerte für Quecksilber im Reingas liegen zur Zeit bei 50 $\mu g/Nm^3$ Hg, die aber ohne Fällungszusatz nur sehr schwierig eingehalten werden können. Sie werden außerdem in absehbarer Zeit durch die Novellierung der TA-Luft weiter gesenkt, so daß eine Lösung der beschriebenen Problematik immer dringender erforderlich wird.

Es stellt sich daher die Aufgabe, ein Fällmittel anzugeben, das unter thermischer Belastung, im Temperaturbereich von 50° C bis 250° C, aus Abgasen und/oder sauren Wäscherabströmen Arsen und Quecksilber ausfällt, wobei die Bildung von Schwefelwasserstoff oder anderer stark reduzierender Stoffe unterdrückt ist. Ferner ist Aufgabe der vorliegenden Erfindung die Angabe eines Verfahrens zur Herstellung des Fällmittels und die Anwendung des Fällmittels in einem Gas-Waschlösungs-System, bei dem Arsen und Quecksilber entweder direkt aus dem heißen Gasstrom oder aus dem 40° C bis 90° C heißen Waschlösungsabstrom gefällt wird, ohne die Bildung von $Hg^0$ zu bewirken. Die Fällmittel, Herstellungs- und Anwendungsverfahren sind so auszulegen, daß sie insbesondere für Abgasreinigungsverfahren mit zweistufigen Waschsystemen mit einer Gipsumsalzstufe anwendbar sind, wobei bei der Herstellung und Anwendung keine negativen Folgewirkungen auf Luft, Wasser und Boden beispielsweise durch Freisetzung gefährlicher Reaktionsprodukte bewirkt wird.

Das erfindungsgemäße Fällmittel mit Thioacetamid zur Ausfällung von Schwermetallen aus Abgasen bzw. Abwässern ist dadurch gekennzeichnet, daß es Thioacetamid ($C_2H_5SN$) in einer 1 - 20%igen wäßrigen Lösung enthält, die zusätzlich eine Puffersubstanz zur Verhinderung des hydrolytischen Selbstzerfalls enthält, wobei die Lösung einen pH-Wert zwischen 5,5 und 9,0 aufweist.

Das erfindungsgemäße Fällmittel hat eine gute Stabilität und bleibt ca. 4 Tage ohne meßbare Zerfallsprodukte stabil. Das Fällmittel ist geeignet, bei einer Anwendung von 1,5 kg/ $m^3$ Wäscherabwasser einer Müllverbrennungsanlage bis zu 99,8 % des enthaltenen gelösten Quecksilbers und bis zu 72 % des enthaltenen Arsens auszufällen, ohne Bildung elementaren Quecksilbers.

Die chemischen Eigenschaften von Thioacetamid sind denen des Acetamids ähnlich, wobei Thioacetamid mesomeriestabilisiert ist, d.h., daß die C-N-Bindung einen partiellen Doppelbindungscharakter hat, wodurch der Schwefel bindungsaktiviert ist.

Gegenüber Mineralsäuren verhält sich Thioacetamid als schwache Base, wobei sich mit konzentrierten Säuren Salze mit mesomeriestabilisierten Kationen bilden, die leicht wieder hydrolytisch gespalten werden.

Durch Zugabe einer Puffersubstanz kann der hydrolytische Selbstzerfall in $NH_3$, $H_2S$ und in Essigsäure verhindert bzw. verlangsamt und eine pH-Stabilisierung im Bereich von pH 5,5 bis 9,0 erreicht werden. Da bei stärkeren Konzentrationen der wäßrigen Lösung die Geschwindigkeit des hydrolytischen Selbstzerfalls erhöht ist, hat sich eine 1 - 20 %ige wäßrige Lösung als besonders lang haltbar herausgestellt. Der Zusatz der Puffersubstanz begünstigt insbesondere unter thermischer Belastung zwischen 50° C und 250° C die Abspaltung von Thioschwefel zur Bindung von Schwermetallen. Die Puffersubstanz führt daher insbesondere in verdünnten wäßrigen Lösungen von Thioacetamid zur wirksamen Arsen- und Quecksilberfällung aus heißen sauren Abgasen und heißen Waschlösungsabströmen von Gaswaschanlagen, wobei das Thioacetamid in der wäßrigen Phase gegen Zerfall stabilisiert ist und auf diese Weise eine längere Haltbarkeit und Lagermöglichkeit z.B. in Tanks, aufweist. Ferner ist sichergestellt, daß bei der Reaktion des Fällmittels in der sauren Waschzone kein $H_2S$ freigesetzt wird.

Bevorzugt ist der Einsatz von Thioacetamid in einer 1 - 10 %igen wäßrigen Lösung, bevorzugt eine 4 - 6 %igen wäßrigen Lösung und ganz besonders bevorzugt einer etwa 5 %igen wäßrigen Lösung. Bei diesen Gewichts-Konzentrationen ist die Zerfallszeit des Thioacetamids bei noch akzeptabler Wirksamkeit klein.

Als Puffersubstanz ist ein Phosphat geeignet, das jedoch in hohen Substanzmengen erforderlich ist.

Alternativ oder zusätzlich weist die Puffersubstanz ein Carbonat auf, das im Handel preiswert erhältlich ist.

Schließlich kann die Puffersubstanz alternativ oder zusätzlich ein Borat aufweisen, das ebenfalls gute Puffereigenschaften hat.

Als Puffersubstanz ist insbesondere ein Hydrogencarbonat bevorzugt, das besonders gute Puffereigenschaften aufweist. Als Hydrogencarbonat kommt bevorzugt Natriumhydrogencarbonat zum Einsatz.

Die Puffersubstanz wird bevorzugt in einer Konzentration von 1 - 20 Gewichtsprozent, bevorzugter 2 - 10 Gewichtsprozent, besonders bevorzugt 3 - 5 Gewichtsprozent eingesetzt.

Die Einstellung des pH-Wertes spielt eine erhebliche Rolle beim Zerfall von Thioacetamid, da dieses sowohl in saurer Lösung (pH 0,5 - 3,5) als auch in alkalischer Lösung (pH 10 - 13,5) schnell unter Bildung von Ammoniak, Schwefelwasserstoff und Essigsäure zerfällt. In der Praxis ist die Einstellung des pH-Wertes auf 7,0 bis 8,5 und insbesondere 8,1 bis 8,2 besonders bevorzugt, da, wie später gezeigt, in diesem Bereich besonders lange Haltbarkeitszeiten erzielt werden. Das Maximum liegt etwa bei pH 8,2.

In einer bevorzugten Ausführungsform kann das erfindungsgemäße Fällmittel neben der Fällung von Arsen und Quecksilber aus Abgasen und Wäscherabströmen auch zur Fällung von Schwermetallen aus Abwässern im gesamten pH-Bereich von pH 0 - 14 eingesetzt werden Es wird Natriumhydroxid bzw. Natronlauge zugesetzt, und zwar bevorzugt in einer Gewichts-Konzentration von 2 - 8 %, bevorzugter 3 - 7 % und besonders bevorzugt 4 - 6 %. Thioacetamid zerfällt unter Einwirkung von Natronlauge in verdünnter wäßriger Lösung binnen 24 Stunden vollständig zu Ammoniak, Natriumhydrogensulfid und Natriumacetat, wodurch Schwermetalle im gesamten pH-Bereich mit hoher Wirksamkeit ausgefällt werden.

Alternativ oder zusätzlich zum Natriumhydroxid kann Natriumsulfit zugesetzt werden, und zwar bevorzugt in einem Gewichtsverhältnis von 0,5 - 1,5 %, bevorzugter 0,8 - 1,2 Gewichtsprozent und besonders bevorzugt 0,9 - 1,1 Gewichtsprozent.

Alternativ oder zusätzlich kann das Fällmittel Natriumdithionit enthalten, und zwar in einem Gewichtsverhältnis von 2,5 - 7,5 %, bevorzugter 3,5 - 6,5 Gewichtsprozent und besonders bevorzugt 4,5 - 5,5 Gewichtsprozent. Die Reaktivität dieses Gemisches beruht einerseits auf der Wirkung des Dithionits und andererseits auf der Wirkung der Zerfallsprodukte des Thioacetamids.

Das erfindungsgemäße Verfahren zur Herstellung des Fällmittels sieht die Herstellung einer wäßrigen Lösung der Puffersubstanz vor, deren pH-Wert kontrolliert und ggf. mit HCl oder NaOH eingestellt wird. Anschließend wird das Thioacetamid in der entsprechenden Menge zugesetzt. Wichtig ist die Zugabe der Puffersubstanz vor der Einrührung des Thioacetamids, um bereits bei der Lösung höhere Wasserstoffionenkonzentrationen zu vermeiden.

Die Temperatur wird bevorzugt beim Zusetzen von Thioacetamid zwischen 15° C und 20° C gehalten. Höhere Temperaturen wirken sich nachteilig aus.

Das erfindungsgemäße Verfahren zur Herstellung eines Fällmittels bevorzugt zur Abwasserbehandlung sieht die Auflösung von Natriumhydroxid in Wasser vor, wobei anschließend ggf. Natriumsulfit und/oder Natriumdithionit zugesetzt werden, und schließlich Thioacetamid zugesetzt wird.

Die Verwendung des erfindungsgemäßen Fällmittels zur Abgasreinigung einer Verbrennungsanlage, die eine Naßwaschvorrichtung aufweist, ist dadurch gekennzeichnet, daß das Fällmittel einem zur Naßwaschvorrichtung geführten Waschwasserzulauf zugesetzt wird. Alternativ bzw. zusätzlich zu dieser Verwendung wird das Fällmittel einem von der Naßwaschvorrichtung stammenden Waschwasserablauf zugesetzt. Da der Ablauf in einigen Naßwaschvorrichtungen zum Zulauf rückgeführt ist, gelangt das Fällmittel in jedem Fall in den Sprühbereich.

Bevorzugt ist im Zulauf eine Mischeinrichtung vorgesehen, die zum Vermischen des Fällmittels mit dem Waschwasser dient. Ebenfalls bevorzugt ist, daß das Fällmittel zu einem im Zulauf bzw. Ablauf enthaltenen Quecksilber-Anteil auf ein Molverhältnis von 8 - 10 : 1, bevorzugter 9 - 11 : 1 und ganz besonders bevorzugt auf etwa 10 : 1 eingestellt wird. Das Verhältnis von 10 : 1 stellt zugleich die Grenzkonzentration dar, bei der noch sehr wenig elementares Quecksilber entsteht. Dieses Molverhältnis sollte daher bei der Anwendung nicht wesentlich überschritten werden.

Weitere Vorteile, Merkmale und Anwendungsmöglichkeiten der vorliegenden Erfindung ergeben sich aus der nachfolgenden Beschreibung von Ausführungsbeispielen in Verbindung mit der Zeichnung.

Fig. 1 zeigt ein Untersuchungsergebnis zum Zerfall von Thioacetamid in Abhängigkeit vom pH-Wert.

Fig. 2 zeigt den Selbstzerfall von Thioacetamid in Abhängigkeit von der Konzentration der Lösung bei verschiedenen pH-Werten.

Fig. 3 zeigt den zeitlichen Verlauf des Selbstzerfalls einer 5 %igen wäßrigen Thioacetamid-Lösung in Abhängigkeit von der Temperatur.

Fig. 4 zeigt eine Vorrichtung zur Eingabe von Thioacetamid in die erste Waschstufe eines Wirbelstromreaktors.

Fig. 5 zeigt eine Meßapparatur zur Bestimmung der Quecksilberbilanz nach einer Fällung in 100° C heißer Waschabstromlösung.

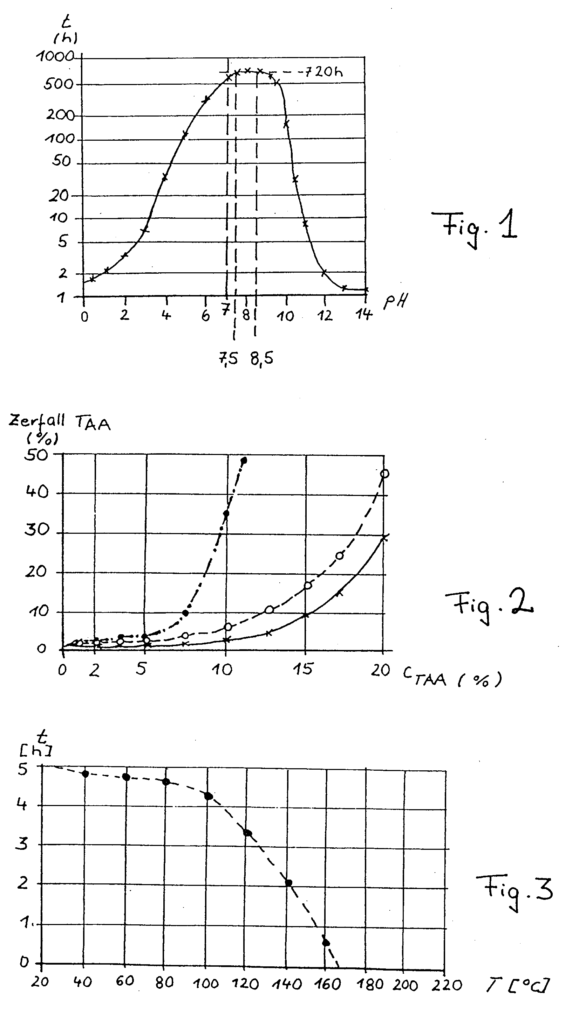

Der in Fig. 1 gezeigten Graphik liegt eine 5 %ige wäßrige Lösung von großtechnisch hergestelltem Thioacetamid für die Versuche zugrunde. Als Meßparameter wurden die Zerfallsprodukte Ammoniak ($NH_3$), Schwefelwasserstoff ($H_2S$) und Essigsäure ($CH_3COOH$) ausgewählt. Zur Einstellung des jeweiligen pH-Wertes wurden verschiedene Puffergemische gewählt.

Das in Fig. 1 gezeigte Untersuchungsergebnis belegt, daß eine wäßrige Lösung von Thioacetamid im pH-Bereich zwischen 7,5 und 8,5 bevorzugt gegen Zerfall stabilisiert ist und mindestens 720 Stunden (30 Tage) lagerstabil bleibt. Die in der Figur dargestellte Zeit t ist die Standzeit bis zum Zerfall von 1 % des enthaltenen Thioacetamid (ursprünglich 50 g/l ≙ 5 Gew.-%), wobei die Temperatur T während der Lagerzeit 20° C beträgt.

Der in Fig. 2 gezeigten Darstellung zum Selbstzerfall von Thioacetamid in Abhängigkeit von der Konzentration der Lösung bei verschiedenen pH-Werten liegt eine Zeit t von 240 Stunden bei einer Temperatur T von 20° C zugrunde. Die einzelnen Kurven beziehen sich auf pH 8,2 (durchgezogene Linie), auf pH 5,2 (gestrichelte Linie), und auf pH 1,0 (strichpunktierte Linie). Aus der Fig. 2 geht hervor, daß bei 5 Gew.-% Thioacetamid die günstigste Konzentration der Wirksubstanz vorliegt. Je nach pH-Wert steigt der Zerfall bei höheren Konzentrationen stark an.

Fig. 3 zeigt den zeitlichen Verlauf des Selbstzerfalls einer 5 %igen wäßrigen Thioacetamid-Lösung in Abhängigkeit von der Temperatur, wobei eine Konzentration des Thioacetamids von 5 Gew.-% und ein pH von 1,0 zugrunde gelegt ist. Die Zeit wird gestoppt bei Zerfall von 5 % des eingesetzten Thioacetamids, wobei sich herausstellt, daß bis etwa 100° C noch eine Beständigkeit bis zu etwa 4 Stunden gegeben ist, und ab 100° C eine linear mit der Temperatur abfallende Beständigkeit bis auf etwa 0,5 h bei 160° C gegeben ist.

Die Resultate der Fig. 2 und 3 zeigen, daß die zur Verbindung der Metalle notwendige Substanzmenge möglichst genau zu dosieren ist, jedoch keinesfalls mit mehr als der 5-fachen Menge überdosiert werden darf, da sonst aus dem Zerfall des unverbrauchten Thioacetamids Schwefelwasserstoff freigesetzt wird, was eine Reduktion des Quecksilbers zu elementarem Quecksilber verursachen kann.

Fig. 3 verdeutlicht zusätzlich die Problematik eines Kontaktes des Thioacetamids direkt in der Sprühzone der Abgaswäsche bei 150° C, da bei dieser Temperatur sehr schnell der Selbstzerfall einsetzt. Da die Kontaktzeit in dieser Zone jedoch kurz ist, muß im großtechnischen Experiment das Optimum zwischen Bindungsgeschwindigkeit, Wirkungsgrad und Selbstzerfall-Minimum des unverbrauchten Thioacetamid-Überschusses bestimmt werden.

Beispiel 1

Nachstehend wird ein Beispiel zur Fertigung von einer Tonne einer 5 %igen wäßrigen Lösung von Thioacetamid mit einem 4 Gew.-%-Gehalt von Natriumhydrogencarbonat beschrieben.

Es wird ein Behälter aus GFK, PP oder Edelstahl eingesetzt, wobei die Rühreinrichtung aus Edelstahl mit mehreren Paddeln besteht, die auf Umdrehungen von 30 bis 180 pro Minute einstellbar sind.

Zum Einsatz kommen Thioacetamid, technisch rein, 99 %, Plättchen und Natriumhydrogencarbonat, technisch rein, Reinheit > 99 % in Pulverform sowie Wasser, entsalzt oder Trinkwasser mit einer Gesamthärte von < 5° dH.

Zu 910 l Wasser werden zunächst 40 kg Natriumhydrogencarbonat zugesetzt und vollständig gelöst. Sodann wird der pH-Wert kontrolliert und ggf. mit HCl oder NaOH auf genau 8,3 eingestellt. Anschließend werden 50 kg Thioacetamid in die gepufferte Lösung eingerührt, wobei die Temperatur zwischen 15° C und 20° C gehalten wird. Nach vollständiger Auflösung des Thioacetamids, ca. 2 Stunden, wird anschließend der pH-Wert kontrolliert und auf pH 8,2 nachgestellt.

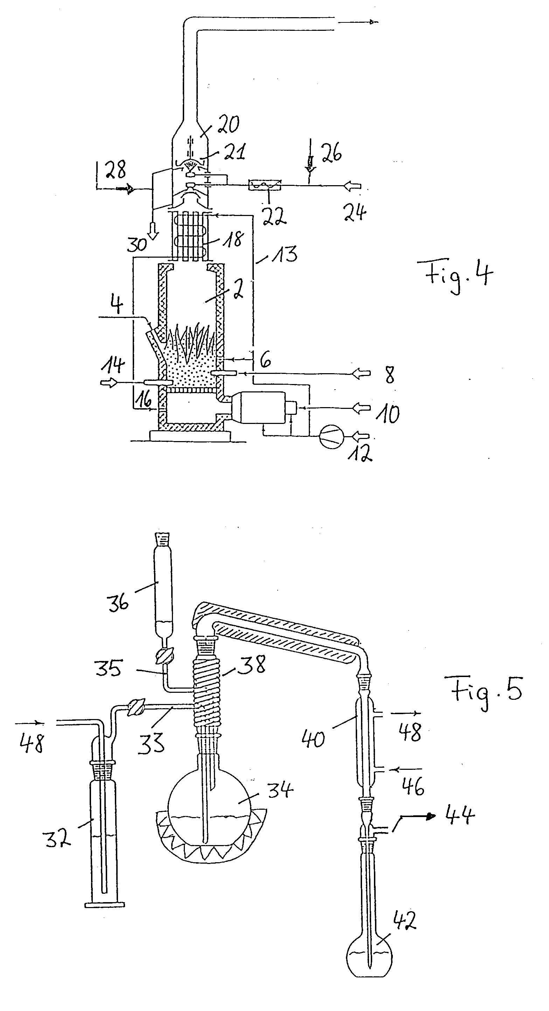

Fig. 4 zeigt eine Vorrichtung zur Eingabe bzw. Anwendung des Fällmittels im Verfahrensgang der Naßreinigung von Verbrennungsanlagen, die nach dem Prinzip der Naßwäsche arbeiten.

Die Vorrichtung zeigt einen Brenner 2, in den ein Brennstoffkanal 10 und ein Luft-Einführkanal 12 geführt sind. Ferner wird ein zu verbrennender Abfallstoff durch einen Kanal 8 in den Brenner eingebracht und ein Zusatzbrennstoff durch einen Kanal 14. Von dem Luftkanal 12 zweigt ein Kanal 13 ab, der zu einem über dem Brenner angeordneten Wärmetauscher 18 geführt ist, welcher die Luft aufheizt und sie durch einen Einlaß 16 in den Brenner führt. Sekundärluft wird durch einen Einlaß 6 eingeführt.

Oberhalb des Wärmetauschers 18 befindet sich eine saure Waschstufe 20, die verschiedene Sprüheinrichtungen 21 aufweist. Die Sprüheinrichtungen 21 sind mit einem statischen Mischer 22 verbunden, der seinerseits mit einer Waschwasser-Zulaufleitung 24 verbunden ist. In die Waschwasser-Zulaufleitung 24 mündet eine Einführleitung 26 für das erfindungsgemäße Fällmittel.

Von den Auffängern der Sprüheinrichtungen 21 führen Waschwasser-Ablaufleitungen weg, die das zu entsorgende Waschwasser in einer Leitung 30 sammeln. In die Waschwasser-Ablaufleitung 30 mündet eine zweite Einführleitung 28 für Thioacetamid-Fällmittel, das erst im Waschwasserablauf zugegeben wird.

Das erfindungsgemäße Fällmittel kann in konzentrierter, jedoch bevorzugt in verdünnter Form dem Waschwasser in der sauren Waschstufe 20 zugesetzt werden, so daß es mit dem Waschwasser zusammen in die Sprüheinrichtung 21 gelangt. Eine Zugabe des Fällmittels in den 50° bis 80° C heißen Waschwasserabstrom ist ebenfalls möglich. Die Zuführleitungen 26 und 28 werden alternativ oder in Kombination je nach den technischen Bedingungen der Anlage verwendet. Die Auslegung ist im technischen Großversuch zu ermitteln.

Wenn das erfindungsgemäße Fällmittel durch die Einführleitung 26 in den Vorlauf eingespritzt wird, wird es in einem statischen Mischer 22 vermischt. Wenn das Fällmittel in den Waschwasserablauf gegeben wird, kann dies in konzentrierter oder verdünnter Form geschehen. Beide Verfahren unterscheiden sich darin, daß zwischen der Gassprühzone und dem ablaufenden Sprühwasserabstrom eine Temperaturdifferenz von ca. 100° C besteht. Im Bereich der Sprühzone liegen Temperaturen zwischen 180° C bis 230° C und im Abstrom 50° C bis 90° C vor.

Die Leistungsfähigkeit des erfindungsgemäßen Thioacetamid-Fällmittels wurde im Laborversuch mit einer in Fig. 5 gezeigten Vorrichtung geprüft.

Die in Fig. 5 gezeigte Vorrichtung weist eine Waschflasche 32 auf, die zur Aufnahme einer KMnO$_4$-Waschlösung für N$_2$ dient, in die ein Rohr 48 zur Einführung von N$_2$ geführt ist. Von der Waschflasche 32 ist eine Leitung 33 zu einem Destillationskolben 34 geführt, der 100 ml saure Wäscherabstromlösung enthält und mit einer Heizvorrichtung versehen ist. Von einem oberen Bereich des Kolbens 34 ist ein Rohr 35 mit Dosierhahn zu einem Behälter 36 mit Thioacetamid-Fällmittel geführt. Eine Destillationsbrücke 38, die zur Aufnahme des Destillats dient, ist mit einer Wärmeisolierung versehen ebenso wie ein Überleitungsrohr, das mit einem Destillationskühler 40 in Verbindung steht. Die Wärmeisolierung der Brücke 38 und des Überleitungsrohrs besteht aus Glaswolle mit Aluminium. Der Destillationskühler 40 ist mit einem Eingangsanschluß 46 und einem Ausgangsanschluß 48 zum Durchlauf von Wasser verbunden, welches zur Kühlung dient. Schließlich ist die Leitung in einen Auffangbehälter 42 für elementares Quecksilber geführt, der mit 50 ml Königswasser befüllt ist. Von diesem zweigt im oberen Bereich ein Stutzen 44 zur Abfuhr von N$_2$ ab. Das N$_2$ drückt somit vom Einlaß 48 bis zum Auslaß 44 und treibt das Destillat in den Behälter 42.

Zur Vorbereitung des Versuchs werden 100 ml des sauren Abstromwassers in den Reaktionskolben 34 eingefüllt. Als Wärmequelle dient ein Heizpilzmantel, der den Reaktionskolben in seinem unteren Bereich umgibt. Die jeweils vorgesehene Menge der 5%igen Fällmittellösung wird in den als Fällmittelvorlage dienenden Behälter 36 eingefüllt. In den als Destillationsvorlage dienenden Auffangbehälter 42 werden 50 ml Königswasser eingegeben, um eventuell entstehendes und austreibbares Hg$^0$ aufzufangen. Die Apparatur wird geschlossen, das Fällmittel aus dem Behälter 36 in den Reaktionskolben 34 eingelassen und sodann werden kontinuierlich 1,5 l/min.N$_2$ durch das Meßsystem gespült.

Der Stickstoff wird vor der Einführung in den Destillationskolben 34 mit saurer KMnO$_4$-Lösung in der Waschflasche 32 gereinigt. Der Reaktionskolben 34 wird zum Sieden erhitzt und 30 ml werden im Verlauf von 45 min in den Auffangbehälter 42 für Königswasser überdestilliert. Anschließend wird der Rückstand des Reaktionskolbens in einen 100 ml-Meßkolben überführt, nach Abkühlung auf exakt 20° C mit Wasser aufgefüllt und danach wird ein Teil über einen Membranfilter (0,5 μm) scharf filtriert.

Der Rückstand und das Filtrat werden aufgeschlossen und die Gesamtmenge Hg sowie die Gelöstmenge Hg bestimmt. Das Destillat wird ebenfalls mit Wasser auf 100 ml ergänzt und der darin enthaltene elementare Anteil des Quecksilbers (Hg$^0$) bestimmt. Mit dieser Anordnung ist die Bestimmung der Gesamtbilanz des Quecksilbers möglich.

In den nachstehend beschriebenen Versuchen 1 bis 3 wird das saure Abstromwasser der ersten Stufe aus einer Müllverbrennungsanlage ohne Zusatz (Versuch 1), mit einem herkömmlichen Polysulfid (Versuch 2) und mit einer 5 %-igen Thioacetamidlösung (Versuch 3) destilliert. Das Abwasser hatte folgende Kenndaten.

| Hg-gesamt | 6740 $\mu$g/l |
|---|---|
| Hg-gelöst | 5330 $\mu$g/l |
| Hg-elementar | 11 $\mu$g/l |
| pH-Wert | 1,4 |

In den Versuchen 5 bis 8 wurde ein höher belastetes Abwasser der gleichen Anlage untersucht, das zudem 184 mg/l Blei (Pb) enthielt. Das Wasser hatte folgende Daten:

| Hg-gesamt | 19500 $\mu$g/l |
|---|---|
| Hg-gelöst | 19200 $\mu$g/l |
| Hg-elementar | 8,5 $\mu$g/l |
| Pb-gesamt | 184 mg/l |
| Pb-gelöst | 183 mg/l |
| pH-Wert | 0,5 |

Die Versuchsergebnisse sind nachfolgend aufgelistet:

Versuch 1

Im ersten Versuch werden 100 ml Wäscherabwasser im Reaktionskolben ohne irgendeinen Zusatz auf 100°C aufgeheizt und mit 1,5 l/min. $N_2$ während einer 45-minütigen Destillation begast.

Sodann wurden nach der zuvor beschriebenen Aufbereitung folgende Hg-Gehalte gemessen:

| Destillationsrückstand: | |
|---|---|
| Hg-gesamt | 6730 $\mu$g/l |
| Hg-gelöst | 5690 $\mu$g/l |

| Destillat: | |
|---|---|
| Hg-elementar | 13,4 $\mu$g/l |

Versuch 2

Im zweiten Versuch werden den 100 ml Abwasser im Reaktionskolben 0,15 g entsprechend 1,5 kg/m$^3$ eines polysulfidischen Fällmittels zugegeben und danach wie bei Versuch 1 verfahren. Es wurden folgende Hg-Gehalte gemessen:

| Destillationsrückstand: | |
|---|---|
| Hg-gesamt | 6030 $\mu$g/l |
| Hg-gelöst | 3405 $\mu$g/l |

| Destillat: | |
|---|---|
| Hg-elementar | 710 $\mu$g/l |

Versuch 3

Im dritten Versuch wurden den 100 ml Abwasser im Reaktionskolben 0,10 g entsprechend 1,0 Kg pro m³ einer 5 %-igen Thiocetamidlösung gemäß der Erfindung zugegeben und danach wie bei Versuch 1 verfahren. Es wurden folgende Hg-Gehalte gemessen:

| Destillationsrückstand: | |
|---|---|
| Hg-gesamt | 6735 $\mu$g/l |
| Hg-gelöst | 240 $\mu$g/l |

| Destillat: | |
|---|---|
| Hg-elementar | < 1 $\mu$g/l |

Versuche 5 bis 8

Bei den Versuchen 5 bis 8 wurde ein höher mit Quecksilber belastetes Abwasser einer Müllverbrennungsanlage (MVA) verwendet. Zudem war dieses Abstromwasser mit erheblichen Bleigehalten beaufschlagt. Mit den Versuchen sollte eine optimale Dosierung ermittelt werden und zusätzlich geprüft werden, ob insbesondere Bleianteile bei dem niedrigen pH-Wert von 0,5 zum Teil mitgefällt werden.

Das Abwasser hatte folgende Ausgangsdaten:

| | |
|---|---|
| Hg-gesamt | 19500 $\mu$g/l |
| Hg-gelöst | 19200 $\mu$g/l |
| Hg-elementar | 8,5 $\mu$g/l |
| Pb-gesamt | 184 mg/l |
| Pb-gelöst | 183 mg/l |
| pH-Wert | 0,5 |

Die Versuche wurden analog zu den Versuchen 1 bis 3 durchgeführt. Lediglich die Zugabemenge der 5 %-igen Thioacetamid-Lösung wurde variiert. Es ergaben sich folgende Ergebnisse.

| Versuch-Nr. | Dosiermenge TAA, 5 % (kg/m³) | Destillationsrückstand | | Destillat Hg⁰ ($\mu$g/l) | Destillationsrück. | |
|---|---|---|---|---|---|---|
| | | Hg-gesamt ($\mu$g/l) | Hg-gelöst ($\mu$g/l) | | Pb-ges. (mg/l) | Pb-gel. (mg/l) |
| 5 | 0,5 | 19500 | 14500 | < 1 | 184 | 183 |
| 6 | 1,0 | 19500 | 8600 | < 1 | 184 | 184 |
| 7 | 1,5 | 19500 | 471 | 1 | 184 | 183 |
| 8 | 2,0 | 19500 | 120 | 1,8 | 184 | 183 |

Versuch 9

Das für den Versuch 9 verwendete Abwasser enthielt kaum Arsen (< 10 $\mu$g/l). Es wurden daher von einer konzentrierten Stammlösung (1 g/l As³⁺) 5 ml zu 1 l des Abwassers aus den Versuchen 5 bis 8 zugegeben. Das Abwasser enthielt danach folgende Konzentrationen:

| Hg-gesamt | 19480 $\mu$g/l |
|---|---|
| Hg-gelöst | 19185 $\mu$g/l |
| Hg-elementar | 8,4 $\mu$g/l |
| As-gesamt | 4980 $\mu$g/l |
| As-gelöst | 4950 $\mu$g/l |

Danach wurde das Abwasser nach der beschriebenen Versuchsanordnung unter Zusatz von 1,5 kg/m$^3$ Thioacetamid-Lösung 5 %-ig behandelt und dann untersucht.

Es wurden folgende Ergebnisse ermittelt:

| Hg-gesamt | 19480 $\mu$g/l |
|---|---|
| Hg-gelöst | 560 $\mu$g/l |
| Hg-elementar | 1 $\mu$g/l |
| As-gesamt | 4980 $\mu$g/l |
| As-gelöst | 1390 $\mu$g/l |

Versuchsergebnisse

a) Versuche 1 bis 3

Der Blindversuch ohne Zugabe von Fällungsmitteln zeigt, daß von dem vorhandenen Quecksilber 21,0 % ungelöst und 78,84 % ionogen gelöst vorliegen. Lediglich 11 $\mu$g/l entsprechend 0,16 % liegen in der elementaren Form als Hg$^0$ vor. Nach der thermischen Belastung (Versuch 1) ohne Fällmittelzugaben verändert sich das Ergebnis ohne prinzipielle Verschiebungen zum Hg$^0$. Der Gehalt an Hg-gesamt bleibt mit 6730 $\mu$g/l nahezu gleich, davon liegen aber 5690 $\mu$g/l entsprechend 84,5 % ionogen gelöst vor. Der elemtare Hg-Anteil steigt geringfügig auf 13,4 $\mu$g/l, entsprechend 0,2 % an. Das Ergebnis des Versuchs mit einem polysulfidischen Fällmittel (Versuch 2) zeigt eine grundsätzliche Veränderung der Verhältnisse. Der Restgehalt an Quecksilber im Destillationsrückstand geht auf 6030 $\mu$g/l entsprechend 89,5 % zurück. Bezogen auf die Ausgangskonzentration liegen hiervon 50,4 % ionogen gelöst und 10,5 % elementar als Hg$^0$ vor, das wiedergefunden wird. Dieses Ergebnis deckt sich weitgehend mit einem großtechnischen Versuch in der Müllverbrennungsanlage, aus der das Abwasser stammt. Das Ergebnis bestätigt zugleich die Realitätsbezogenheit der Laborversuchsanordnung. Das Ergebnis des Versuchs 3 mit der 5 %-igen Thioacetamid-Lösung zeigt eine fast vollständige Rückhaltung des Quecksilbers (6735 $\mu$g/l), wovon lediglich noch 240 $\mu$g/l entsprechend 3,56 % gelöst vorliegen. Der elementare Quecksilberanteil beträgt 1 $\mu$g/l im Destillat.

b) Versuche 5 bis 9

Die Ergebnisse der Versuche 5 bis 8 bestätigen im Prinzip die Ergebnisse des Versuchs 3. Die Anwendung von 1,5 kg/m$^3$ einer 5 %-igen Thioacetamidlösung zeigt mit 97,6 % Fällwirkung (Versuch 7) ein vertretbares Ergebnis. Die gefundenen 1 $\mu$g/l elementares Quecksilber im Destillat deuten jedoch an, daß das für die praktische Anwendung gefundene optimale Molverhältnis von Thioacetamid zu Quecksilber (TAA: Hg) von 10:1 zugleich die Grenzkonzentration darstellt, bei der der noch sehr wenig Hg$^0$ entsteht. Dieses molare Anwendungsverhältnis sollte daher nicht wesentlich überschritten werden.

Das Ergebnis des Versuches 9 zeigt neben der gleichbleibenden Bleikonzentration unabhängig von der Dosierung eine fast gleich gute Hg-Fällung (97,1 %), ebenso wie der Versuch 7. Zusätzlich wurden 72,1 % des als Standard zudosierten Arsens ebenfalls gefällt. Anschließende Neutralisationsversuche zeigten, daß bereits ab pH 2,8 andere Schwermetalle durch die restlichen Überschüsse des thermisch gespaltenen Thioacetamids gefällt werden. In Folge dessen kann kein überschüssiges Fällmittel aus der der sauren Wäsche nachgeschalteten Abwasserneutralisation ausgetragen werden.

Nachstehend folgt eine ökotoxikologische Betrachtung des Zusatzes von Thioacetamid im Abgas und in den sauren Wäscherabströmen von Gasreinigungsanlagen.

Eine Dosierung von 1,5 kg/m$^3$ einer 5 %-igen Thioacetamid-Lösung entsprechen einem Stoffeinsatz von

$$1500 * 0,05 = 75 \text{ g/m}^3$$

Diese Stoffmenge, die fast exakt ein Mol des Stoffes je m$^3$ Abwasser ist, erzeugt bei totalem Verbrauch des Schwefelanteils keine freien Restmengen von Thioacetamid, die über die Neutralisationsanlage hinaus in ein Gewässer oder eine komunale Abwasserreinigungsanlage gelangen könnten.

Bei dem Verbrauch der Substanz werden theoretisch als Folgestoffe Essigsäure bzw. Acetat und Ammoniak-Stickstoff freigesetzt, die beide aus ökotoxikologischer Sicht nur sehr geringe Giftigkeitspotentiale beinhalten.

Es entstehen bei dem vorgestellten Beispiel maximal 60 mg/l Acetat und 14 mg/l Ammoniak-Stickstoff, die zugleich die Obergrenze darstellen, da in der Regel die Quecksilbergehalte in Abwässern von Müllverbrennungsanlagen und Kraftwerken meist nur im Bereich von etwa 5 mg/l liegen.

Laboruntersuchungen bestätigen, daß ausschließlich die genannten Folgestoffe Essigsäure/Acetat und Ammoniakstickstoff (NH$_3$-N) gebildet werden und kein freies Thioacetamid aus einer nachgeschalteten Neutralisationsstufe herausgeht. Allerdings ergaben die Messungen auch, daß von den theoretisch errechneten 60 mg/l Essigsäure oder ihren Salzen nur 8 mg/l nach einer Kalkmilchneutralisation in der klaren Wasserphase gemessen werden können. Von den restlichen 52 mg/l konnten 48 mg/l nach Destillation des Kalk-Gipsschlammes der Neutralisation wiedergefunden werden, so daß eine Bestätigung über eine näherungsweise aufgehende Bilanz gegeben ist. Von den theoretisch freisetzbaren NH$_3$-N Konzentrationen wurden nur 2,2 mg/l im Klarwasser nach der Neutralisation wiedergefunden.

Da die Neutralisation nur bis pH 8,5 stattfindet, sind keine gasförmigen NH$_3$-Verluste erklärbar. Erklärbar ist jedoch die Annahme, daß ein Teil der thermisch abgespaltenen NH$_2$-Gruppe mit NO$_x$-Verbindungen im Abgas unter Bildung von N$_2$ reagiert hat. Als relevante Folgebelastungen aus der Anwendung von Thioacetamid zur Reinigung von Rauchgasen sind also maximal 9 mg/l CSB und 2,2 mg/l NH$_3$-N am Ausgang einer Neutralisationsanlage zu erwarten.

Beispiel 2

Im folgenden wird die Herstellung eines Fällmittels zur Ausfällung von Schwermetallen aus Abwässern von Verbrennungsanlagen und Müllverbrennungsanlagen sowie aus anderen schwermetallbelasteten Abwässern beschrieben.

Das vorstehend beschriebene Fällmittel zur Ausfällung insbesondere von Arsen und Quecksilber aus Abgasen und Wäscherabströmen ändert seine Eigenschaften, wenn weitere Zusatzstoffe, insbesondere Alkalilauge, hier bevorzugt Natronlauge (NaOH) zugesetzt werden. Nachstehend wird ein Fällmittel beschrieben, das im gesamten pH-Bereich von pH 0 bis 14 des Rezipienten (Abwasser u.a.) Schwermetalle ausfällt. Dieses Fällmittel eignet sich jedoch nicht für Abgase.

Zusammensetzung des erfindungsgemäßen Fällmittels:
50 kg Natriumhyperoxid, Perlen, fest
10 kg Natriumsulfit
50 kg Natriumdithionit
50 kg Thioacetamid
840 l entsalztes Wasser
Zur Herstellung des erfindungsgemäßen Fällmittels werden zu 840 l entsalztem Wasser nacheinander 50 kg NaOH-Perlen, nach deren Lösung 10 kg Natriumsulfit, nach dessen Auflösung 50 kg Natriumditionit und zum Schluß 50 kg Thioacetamid zugesetzt. Die Lösung muß nach vollständiger Auflösung aller Komponenten (ca. 3 Stunden) weitere 24 Stunden ausreagieren. Nach dieser Zeit hat die Lösung einen deutlichen Ammoniakgeruch, wodurch die erforderliche Ausreaktion angezeigt wird. Die Reaktivität der Mischung beruht einerseits auf der Wirkung des Dithionits verbunden mit den Zerfallsprodukten des Thioacetamids. Thioacetamid zerfällt unter Einwirkung von Natronlauge in verdünnter wäßriger Lösung binnen 24 Stunden vollständig in Ammoniak, Natriumhydrogensulfid und Natriumacetat. Als Folge hiervon bildet sich ein Reaktionsgemisch, das geeignet ist, aus Abwässern Schwermetalle zwischen pH 0 und 14 mit hoher Effektivität auszufällen.

**Patentansprüche**

1. Fällmittel mit Thioacetamid zur Ausfällung von Schwermetallen aus Abgasen bzw. Abwässern, dadurch gekennzeichnet, daß es Thioacetamid (C$_2$H$_5$SN) in einer 1-20 %igen wäßrigen Lösung enthält, die zusätzlich eine Puffersubstanz zur Verhinderung des hydrolytischen Selbstzerfalls enthält, wobei die Lösung einen pH-Wert zwischen 5,5 und 9,0 aufweist.

2. Fällmittel nach Anspruch 1, dadurch gekennzeichnet, daß Thioacetamid in einer 1 - 10 %igen wäßrigen Lösung, bevorzugt 4 - 6 %igen wäßrige Lösung und besonders bevorzugt einer etwa 5 %igen wäßrigen Lösung vorliegt.

3. Fällmittel nach Anspruch 1, dadurch gekennzeichnet, daß die Puffersubstanz ein Phosphat aufweist.

4. Fällmittel nach Anspruch 1, dadurch gekennzeichnet, daß die Puffersubstanz ein Carbonat aufweist.

5. Fällmittel nach Anspruch 1, dadurch gekennzeichnet, daß die Puffersubstanz ein Borat aufweist.

6. Fällmittel nach Anspruch 4, dadurch gekennzeichnet, daß die Puffersubstanz ein Hydrogencarbonat aufweist.

7. Fällmittel nach Anspruch 6, dadurch gekennzeichnet, daß die Puffersubstanz Natriumhydrogencarbonat (NaHCO$_3$) aufweist.

8. Fällmittel nach einem der vorhergehenden Ansprüche, dadurch gekennzeichnet, daß die Puffersubstanz in einer Konzentration von 1 - 20 Gew.-%, bevorzugt 2 - 10 Gew.-%, besonders bevorzugt 3 - 5 Gew.-% in der Lösung vorliegt.

9. Fällmittel nach einem der vorhergehenden Ansprüche, dadurch gekennzeichnet, daß es einen pH-Wert von 7,0 - 8,5, bevorzugt 8,1 - 8,3 und besonders bevorzugt etwa 8,2 hat.

10. Fällmittel nach Anspruch 1, insbesondere zur Abwasserbehandlung, dadurch gekennzeichnet, daß es Natriumhydroxid (NaOH) enthält.

11. Fällmittel nach Anspruch 10, dadurch gekennzeichnet, daß es Natriumhydroxid in einer Konzentration von 2 - 8 Gew.-%, bevorzugt 3 - 7 Gew.-% und besonders bevorzugt 4 - 6 Gew.-% enthält.

12. Fällmittel nach Anspruch 1 oder 10, dadurch gekennzeichnet, daß es Natriumsulfit enthält.

13. Fällmittel nach Anspruch 12, dadurch gekennzeichnet, daß Natriumsulfit in einem Gewichtsverhältnis von 0,5 - 1,5 Gew.-%, bevorzugt 0,8 - 1,2 Gew.-% und besonders bevorzugt 0,9 - 1,1 Gew.-% vorliegt.

14. Fällmittel nach einem der Ansprüche 1, 10 oder 12, dadurch gekennzeichnet, daß es Natriumdithionit enthält.

15. Fällmittel nach Anspruch 14, dadurch gekennzeichnet, daß es Natriumdithionit in einem Gewichtsverhältnis von 2,5 - 7,5 Gew.-%, bevorzugt 3,5 - 6,5 Gew.-%, besonders bevorzugt 4,5 - 5,5 Gew.-% enthält.

16. Verfahren zur Herstellung eines Fällmittels nach einem der Ansprüche 1 - 9, dadurch gekennzeichnet, daß eine wäßrige Lösung der Puffersubstanz hergestellt wird, daß der pH-Wert der Lösung eingestellt wird und daß Thioacetamid zugesetzt wird.

17. Verfahren nach Anspruch 16, dadurch gekennzeichnet, daß die Temperatur beim Zusetzen von Thioacetamid zwischen 15° C und 20° C gehalten wird.

18. Verfahren zur Herstellung eines Fällmittels nach einem der Ansprüche 10 - 15, dadurch gekennzeichnet, daß Natriumhydroxid in Wasser aufgelöst wird, daß anschließend die Puffersubstanz und ggf. Natriumsulfit und/oder Natriumdithionit zugesetzt wird, und daß schließlich Thioacetamid zugesetzt wird.

19. Verwendung des Fällmittels gemäß einem der Ansprüche 1 bis 15 zur Abgasreinigung einer Verbrennungsanlage, die eine Naßwaschvorrichtung aufweist, dadurch gekennzeichnet, daß das Fällmittel einem zur Naßwaschvorrichtung geführten Waschwasserzulauf zugesetzt wird.

20. Verwendung des Fällmittels gemäß einem der Ansprüche 1 bis 15 zur Abgasreinigung einer Verbrennungsanlage, die eine Naßwaschvorrichtung aufweist, dadurch gekennzeichnet, daß das Fällmittel einem von der Naßwaschvorrichtung stammenden Waschwasserablauf zugesetzt wird.

21. Verwendung nach Anspruch 19, dadurch gekennzeichnet, daß im Zulauf eine Mischeinrichtung vorgesehen wird, die zum Vermischen des Fällmittels mit dem Waschwasser dient.

Fig. 1

Fig. 2

Fig. 3

Fig.4

Fig.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,A | EP-A-0 333 672 (CASTELLINI S.P.A.) <br> * Spalte 2, Zeile 10 - Zeile 20 * <br> * Spalte 3, Zeile 5 - Zeile 56 * <br> --- | 1 | C02F1/52 |
| D,A | DE-A-26 28 649 (CHEMISCHES INSTITUT SCHÄFER AG) <br> * Seite 19; Ansprüche 1,2,5 * <br> * Seite 7, Zeile 11 - Seite 8, Zeile 1 * <br> --- | 1,3 | |
| A | DE-U-90 07 301 (STRATEN, GÜNTER) <br><br> * Seite 12; Ansprüche * <br> ----- | 1,10,12, 14 | |

**RECHERCHIERTE SACHGEBIETE (Int.Cl.5)**

C02F
B01D

Der vorliegende Recherchenbericht wurde für alle Patentansprüche erstellt

| Recherchenort | Abschlußdatum der Recherche | Prüfer |
|---|---|---|
| DEN HAAG | 11. Mai 1994 | Teply, J |

EPO FORM 1503 03.82 (P04C03)

**KATEGORIE DER GENANNTEN DOKUMENTE**

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
 
& : Mitglied der gleichen Patentfamilie, übereinstimmendes
    Dokument